# EUROPÄISCHE PATENTANMELDUNG

(11) **EP 1 097 943 A1**
(43) Veröffentlichungstag der Anmeldung: **09.05.2001**
(21) Anmeldenummer: 01101484.2
(22) Anmeldetag: 13.05.1995
(51) Int. Cl.: C07K 14/805, C07K 1/36

(54) **Verfahren zum Herstellung molekular - einheitlicher hyperpolymer Hämoglobine**

(30) Priorität: 31.05.1994 DE 4418973
(62) Teilanmeldung aus: 95107280.0
(71) Anmelder: SanguiBioTech AG, 58455 Witten an der Ruhr (DE)
(72) Erfinder: Barnikol, Wolfgang, Prof. Dr. Dr., 55128 Mainz (DE)
(74) Vertreter: Beil, Hans Christoph, Dr.

(57) **Zusammenfassung**

Die Erfindung betrifft ein Verfahren zur Herstellung molekular-einheitlicher hyperpolymerer Hämoglobine, bei dem eine an sich bekannte Lösung hyperpolymerer Hämoglobine mit unterschiedlichen Molekulargewichten wenigstens einem Ultrafiltrationsprozeß und/oder wenigstens einem fraktionierten Fällungsprozeß und/oder wenigstens einem Chromatographieprozeß und/oder wenigstens einem partiellen Lösungsprozeß unterworfen wird.

## Beschreibung

Die Erfindung betrifft ein Verfahren zur Herstellung molekular-einheitlicher hyperpolymerer Hämoglobine.

Chemische Modifikationen von Hämoglobinen, z.B. Veränderungen der Sauerstoff-Affinität oder Polymerisation des Moleküls, werden vorgenommen, um sogenannte Blutersatzmedien bereitzustellen, die beim Menschen die Sauerstoff-Transport-Funktion unterstützen können.

Diese Blutersatzmedien können beispielsweise bei einem Verkehrsunfall mit schwer zu beherrschenden Blutungen, bei Unfällen unter Blutverlust oder für den Fall eines Infektionsrisikos (Hepatitis, AIDS) als Ersatz für eine momentan nicht verfügbare, passende Blutkonserve infundiert werden; dies gilt auch dann, wenn ein Mensch, z.B. in den genannten Fällen in einen Volumen-Mangel-Schock geraten ist. Es ist möglich, daß eine sauerstoffübertragende Blutersatzlösung einen Volumen-Mangel-Schock eher durchbrechen kann als eine Blutkonserve, da die Erythrozyten bekanntlich in der Konserve versteift sind und dadurch eine verringerte Kapillardurchgängigkeit aufweisen. Ein sauerstofftransportierender Blutersatz ist gegenüber einer Blutkonserve auch für den Fall günstiger, wenn die Gefahr einer immunologischen Überreaktion besteht. An Tierversuchen ist gezeigt worden, daß mit sauerstoffübertragenden Blutersatzlösungen ein Volumen-Mangel-Schock wirksamer bekämpft werden kann als mit einfachen Plasmaexpandern (Übersichtsartikel hierzu: R.Pabst, Med. Klin. 72 (1977), 1555-1562). Es ist ferner zu erwarten, daß auch chronische Durchblutungsstörungen (beispielsweise koronare, cerebrale und periphere) mit Hilfe geeigneter Polyhämoglobinlösungen wirksam bekämpft werden können. Nicht zuletzt lassen sich Sauerstoff-Mangelzustände ohne Durchblutungsverminderung z.B. chronische Anämien, mit solchen Lösungen bekämpfen. Diese Indikation besitzt schätzungsweise sogar einen zehnfach größeren Anwendungsbereich.

Zur Herstellung sauerstoffübertragender Blutersatzmedien sind bereits verschiedene Wege beschritten worden, nämlich
1. Verwendung von Emulsionen von Fluorkohlenwasserstoffen, in welchen der Sauerstoff sehr gut löslich ist (Übersichtsartikel hierzu: Hirlinger et al., Anästhesist 31 (1982), 660-666). Diese Methode hat jedoch den Nachteil, daß bei Anwendung von Fluorkohlenwasserstoffen gewebliche Reaktionen auftreten.
2. Die Mikroverkapselung konzentrierter Hämoglobinlösungen in Phospholipid-Vesikeln zu sogenannten "künstlichen Erythrozyten" (Übersichtsartikel hierzu: Gaber et al., Encapsulation of hemoglobin in Phospholipid Vesicles; Preparation and Properties of a Red Cell Surrogate in "The Red Cell Sixth Ann Arbor Conference", G.J. Brewer (Herausgeber), Alan R. Liss, Inc. New York, (1984), 179-190). Diese Methode befindet sich allerdings derzeit noch im Entwicklungsstadium der Tierversuche. Außerdem besteht hier die Gefahr einer Lipoid-Überbelastung des Organismus durch vesikelbildende Lipoide.
3. Herstellung geeigneter Hämoglobinlösungen. Diese Methode bietet die besten Erfolgsaussichten.

Die DE-OS 24 17 619 beschreibt beispielsweise polymerisiertes, verknüpftes Hämoglobin als Plasmaprotein-Ersatz, wobei Dicarboxilidat-verknüpftes Hämoglobin hergestellt wird.

Die DE-OS 27 14 252 beschreibt Pyridoxalphosphat-verknüpftes Hämoglobin.

Die DE-OS 30 29 307 betrifft ein Blutersatzmittel, das durch kovalente Verknüpfung von Polysaccharid, beispielsweise Dextran, mit zellfreiem Hämoglobin hergestellt wird.

Die BE-PS 838 933 beschreibt die Herstellung eines wasserlöslichen, verknüpften, polymerisierten Hämoglobins durch Umsetzung freien Hämoglobins mit einem polyfunktionellen verknüpfenden Agens und anschließendem Abstoppen der Reaktion mit einem inaktivierenden Mittel. Es wird eine polymeres Hämoglobin mit einem Molekulargewicht von 64 000 bis 1 000 000 Dalton erhalten.

Die US-PS 40 01 401 betrifft ein verknüpftes, polymerisiertes Hämoglobin als Blutersatz und Plasmaexpander mit einem Molekulargewicht von 64 000 bis 1 000 000 Dalton, das mittels der verknüpfenden Agenzien Glutaraldehyd, Hexamethylendiisocyanat oder Butadiendiepoxid gewonnen wird.

Die EP-PS 0 201 618 betrifft ein Verfahren, aus hoch konzentrierten Lösungen von monomerem Hämoglobin extrem hochmolekulare, kompakte, lösliche Polymere, sogenannte Hyperpolymere, des Hämoglobins herzustellen.

In der DE-PS 37 14 351 ist dieses Verfahren insofern vereinfacht, als direkt Eryrthrozyten Verwendung finden können, und das Vernetzungsmittel nicht mehr in einer Lipid-Phase zugesetzt werden muß.

Bei der Präparation geeigneter modifizierter Hämoglobin-Lösungen für eine routinemäßige, klinisch-praktische Nutzung tritt unter anderem die Notwendigkeit auf, die Viskosität der Lösung möglichst klein zu halten. Die Viskosität des Blutes bestimmt entscheidend den sogenannten totalen peripheren Widerstand des Organismus mit; jener darf keinesfalls zu groß sein, weil das der Kreislauf nicht toleriert.
Im Fall von Polymerlösungen , und das gilt speziell auch für Hämoglobinpolymere , treten solche Probleme vor allem dann auf, wenn eine Polymerisation zu Kettenmolekülen erfolgt. Damit die Viskosität der Lösungen klein bleibt, sollte das Polymer möglichst kompakt sein und kein durchspültes Fadenmolekül, um bei möglichst geringer Viskosität des Plasmas eine möglichst große Konzentration des Sauerstoffträgers applizieren zu können.
Das Einsteinsche Viskositätsgesetz besagt, daß einheitlich große Kugeln in einer Flüssigkeit, und zwar unabhängig von ihrem Radius, eine minimale Viskosität aufweisen.

Daher wäre es zur Verringerung der Viskosität extrem wichtig, möglichst einheitliche sauerstofftragende Moleküle, wie sie im übrigen auch in der Natur (Regenwurm) zu finden sind, herstellen zu können. Dann könnte das Molekulargewicht des Sauerstoffträgers sehr hoch gemacht werden, damit andererseits die Forderung nach einem vernachlässigbaren kolloidosmotischen Druck erfüllt ist.

Für den Fall eines sogenannten hypokotischen Blutzusatzes auf der Basis von Hämoglobinlösungen besteht außerdem die Notwendigkeit, speziell niedermolekulare Anteile der Hyperpolymeren unbedingt zu entfernen.

Mit der EP-PS 0 201 618 und der DE-PS 37 14 351 kann das Problem der Verknüpfung von Hämoglobin zu kompakten aber löslichen Riesenmolekülen als gelöst angesehen werden. Die dort beschriebenen Verfahren führen jedoch zu einem Hyperpolymergemisch mit einer breiten Verteilung des Molekulargewichts und einer stark überproportionalen Zunahme der Viskosität bei steigender Konzentration (Barnikol and Burkhard, Adv. Exp. Biol. Med. 248 (1989) 335-340).

Aufgabe der vorliegenden Erfindung ist es deshalb, ein Verfahren zu schaffen, mit dem aus einer bekannten Hämoglobin-Hyperpolymer-Lösung mit Hyperpolymeren unterschiedlicher Molekulargewichte molekular-einheitliche Hämoglobin-Hyperpolymere abgetrennt werden können.

Eine Lösung dieser Aufgabe besteht darin, eine an sich bekannte Lösung hyperpolymerer Hämoglobine unterschiedlichen Molekulargewichts wenigstens einem Ultrafiltrationsprozeß und/oder wenigstens einem fraktionierten Fällungsprozeß und/oder wenigstens einem Chromatographie-Prozeß und/oder wenigstens einem partiellen Lösungsprozeß zu unterwerfen.

Die Erfindung umfaßt nicht einen Ultrafiltrationsprozeß unter Verwendung eines Filters mit einer normalen Trenngrenze von 10⁶g/mol wobei hyperpolymere Hämoglobine in einem Endmolekulargewichtsbereich von 500 000- 15x10⁶ g/mol erhalten werden und die an sich bekannte Lösung hyperpolymere Hämoglobine unterschiedlichen Molekulargewichts ein Ausgangsmolekulargewicht von 65 000 bis 15x10⁶ aufweisen.

Die genannten Methoden sind im Stand der Technik als Verfahren zur molekulargewichtsabhängigen Abtrennung von solchen Proteinen bekannt, deren Größe bzw. Gewicht maximal durch eine Quartärstruktur bestimmt ist. Der oberere Molekulargewichtsbereich liegt nach bisherigen Erfahrungen bei etwa 500 000. Dies ist auch die obere Grenze der im Handel erhältlichen Markerproteinen zur vergleichenden Molekulargewichtsbestimmung. Bei besonders großen Proteinen besteht bekanntermaßen das Problem, daß diese oft nicht als Ganzes abgetrennt werden, sondern aus bisher größtenteils noch unbekannten Gründen ganz oder teilweise zerfallen, und man infolgedessen nur Untereinheiten abgetrennt erhält.

Bei den der Erfindung zugrundeliegenden Hämoglobin-Hyperpolymeren handelt es sich im Unterschied zu den gewöhnlich vorliegenden Proteinen um Riesenmoleküle, die erst in jüngster Zeit erstmals synthetisiert wurden, und deren Größe bzw. Gewicht -je nach dem Polymerisationsgrad- ein Hundert- bis Mehrhundertfaches des an sich schon relativ großen und schweren quartärstrukturierten Hämoglobin-Moleküls beträgt.

Es wurde nun überraschenderweise gefunden, daß mit den an sich bekannten Verfahren der Ultrafiltration, fraktionierten Fällung, Chromatographie und des partiellen Lösens auch aus einem Gemisch solcher hyperpolymeren Hämoglobin- Riesenmoleküle Fraktionen mit einheitlichem Molekulargewicht abgetrennt werden können. Bei diesen Hämoglobin- Rieseomoleküle handelt es sich nach ersten und vorläufigen polymeranalytischen Studien zur Viskosität, Lichtstreuung und Ultrazentrifugation (Pötschke, Barnikol, Biol Chem. Hoppe-Seyler, 373 (1992) 811; und Massenkeil, Kirste, Pötschke, Barnikol, Biol. Chem. Hoppe- Seyler 373 (1992) 798) um Kettenmoleküle. Hinsichtlich einer Ultrafiltration war bei solchen Riesenmolekülen aufgrund des allgemeinen Kenntnisstands viel eher zu erwarten gewesen, daß eine Passagierung durch die im wesentlichen kreisförmigen Poren eines Ultrafilters schon aufgrund des Kettencharakters der Hämoglobin-Hyperpolymere zu keiner molekulargewichtsspezifischen Abtrennung führt. Dafür sprach auch, daß es bisher nicht gelang, die hochmolekularen Anteile im Filtrat zu entfernen. Entgegen jener Erwartung wurde jedoch überraschenderweise gefunden, daß es mit Hilfe der Ultrafiltration möglich ist, aus einem Rohpolymerisat mit breitem Molekulargewichtsbereich die niedermolekularen Anteile im Retentat zu entfernen. Damit geht als weiterer Vorteil einher, daß der kolloidosmotische Druck des Polymerisates entscheidend gesenkt wird.

Da die Bereitstellung von Hämoglobin-Hyperpolymeren erst in jünster Zeit gelungen ist, gibt es grundsätzlich bislang noch wenig Erfahrungen bezüglich der physikalischen und chemischen Eigenschaften dieser Riesenmoleküle. Infolgedessen war auch ihr Verhalten bei einem Fällungsprozeß nicht vorhersehbar und-die gefundene Eignung dieses Verfahrens ein überraschendes Ergebnis. Mit Hilfe einer fraktionierten Fällung lassen sich aus einem Hämoglobin-Hyperpolymerisat mit breitem Molekulargewichtsbereich beispielsweise die hochmolekularen Anteile entfernen.

Auch auf dem Gebiet der Chromatographie gibt es bislang keine Erfahrungen im Umgang mit solchen Riesenmolekülen wie den Hämoglobin-Hyperpolymeren. Aufgrund der Größe und des Kettencharakters war mit dieser Methode eine erfolgreiche, molekulargewichtsspezifische Trennung keinesfalls zu erwarten gewesen. Überraschenderweise wurde aber gefunden, daß sich mittels chromatographischer Verfahren einheitliche hyperpolymere Hämoglobine verschiedenen Molekulargewichts gewinnen lassen.

Das an sich bekannte Verfahren, durch zügiges Waschen eines frisch vernetzten und zunächst noch unlöslichen Hämoglobin-Hyperpolymerisats überflüssige Reaktanten sowie monomere und oligomere Hämoglobin-Molekülen zu entfernen, kann mit den übrigen vorgenannten Verfahren problemlos kombiniert werden. Es können somit auf unerwartet einfache Art und Weise Hämoglobin-Hyperpolymerisate gewonnen werden, deren Bestandteile hinsichtlich ihres Molekulargewichts ein Höchstmaß an Einheitlichkeit aufweisen.

Eine vorteilhafte Ausführung des erfindungsgemäßen Verfahrens sieht vor, daß bei dem bzw. den Ultrafilrationsprozeß (en) verschiedene Filtertypen verwendet werden, und/oder eine Mehrfachfiltration stattfindet, und/oder der pH-Wert geändert wird, und/oder in verschiedenen Konzentrationen ultrafiltriert wird, und/oder die Zusammensetzung des Lösungsmittels variiert wird, und/oder die Temperatur variiert wird, und/oder der transmembranöse Druck und/oder der tangentiale (Über-)Fluß variiert wird.

Bei dem bzw. den fraktionierten Fällungsprozeß(en) können vorteilhafterweise verschiedene Agentien verwendet werden, und/oder die Fällung bei verschiedenen Temperaturen erfolgen, und/oder der pH-Wert variiert werden, und/oder unterschiedliche Lösungsmittel für die zu fällenden polymeren Hämoglobine eingesetzt werden, und/oder die Reaktionszeiten variiert werden und/oder unterschiedlich gesättigte Fällungsmittel verwendet werden.

In einer bevorzugten Ausführungsform werden bei dem bzw. den Chromatographie-prozeß(en) verschiedene chromatographische Verfahren, insbesondere Ionenaustausch- und Gelfiltrationschromatographie, kombiniert oder sukzessiv angewendet, und/oder der pH-Wert geändert, und/oder die Zusammensetzung des Fließmittels verschieden gewählt, und/oder die Chromatographiebedingungen, insbesonder der Fluß und die Beladung, variiert, und/oder verschiedene Chromatographiematerialien eingesetzt.

Bei dem bzw den partiellen Lösungsprozeß(en) ist vorzugsweise vorgesehen, daß die Lösungszeit variiert wird, und/oder wenigstens eine Waschung durchgeführt wird, und/oder die sich lösenden Polymere mit stabilisierenden Agenzien, welche einen Abbau der Polymere insbesondere durch Oxidation verhindern, behandelt werden, und/oder die Lösungsbedingungen, insbesondere die Temperatur und/oder die Menge des Lösungsmittels, geändert werden, und/oder die Zusammensetzung des Lösungsmittels variiert wird.

Durch die Variation der verschiedenen Parameter ist es möglich, jeden der genannten Prozesse auf die Art (unterschiedlice Vernetzer und/oder unterschiedliche Hämoglobine, z.B. Rinder-, Schweine- Menschenhämoglobin) des jeweils vorliegenden und zu trennenden Hämoglobin-Hyperpolymer-Gemisches abzustimmen, und auf diese Weise eine vorteilhaft hohe Trennwirkung für Hämoglobine mit einem Molekulargewicht innerhalb sehr enger Bereichsgrenzen zu erzielen.
Eine Kombination der verschiedenen Trennprozesse untereinander ermöglicht - auch mit Rücksicht auf die unterschiedliche Stabilität der verschiedenen hyperpolymeren Hämoglobine - die Herstellung von Hämoglobin-Hyperpolymeren, deren Molekulargewicht ein Höchstmaß an Einheitlichkeit aufweist.

Die Erfindung wird im folgenden anhand von Ausführungsbeispielen näher erläutert.

### Beispiel 1 (Ultrafiltration)

Menschliches Hämoglobin wurde gemäß bekannter Vorschrift (Pötzschke, Barnikol, Biomater, Art Cells and Immob. Biotechn. 20 (1992) 287-291) mit Hilfe von Glutardialdehyd zu Hyperpolymeren vernetzt. Eine etwa 20%ige Lösung des Hyperpolymeren wurde sodann in der Elektrolytlösung BIKU (in mmol/l: NaCl 125; KCl 4,5; NahCO₃ 20; 0,2 g/l NaN₃) einer Ultrafiltration unterzogen. Dabei fand ein Filter mit einer normalen Trenngrenze von 10⁶ g/mol Verwendung. Der Filtrationsprozeß erfolgte mit dem ungefähr zwanzigfachen Flüssigkeitsvolumen der ursprünglichen Lösung.

Mit Sephacryl S-400 HR (Deutsche Pharmacia, Freiburg/D) lassen sich die hyperpolymeren Hämoglobine bezüglich ihrer Polymolarität analysieren. Die Grenz-Molekulargewichte des unfiltrierten Produkts waren 65 000 (unten) und 15 x 10⁶ g/mol (oben), nach der Ultrafiltration betrugen die des Retentates 500 000 bzw 15 x 10⁶ g/mol. Mit Hilfe der Ultrafiltration ist es somit gelungen, aus dem Rohpolymerisat, dessen Bestandteile einen sehr breiten Molekulargewichtsbereich abdecken, die niedermolekularen Anteile zu entfernen und damit den kolloidosmotischen Druck des Polymerisates entscheidend zu senken.

### Beispiel 2 (fraktionierte Fällung)

Rinderhämoglobin wurde mit dem bifunktionellen Vernetzer 2,5-Diisothiocyanatbenzenesulfonat (DIBS) nach bekannten Verfahren (W.K.R. Barnikol, Adv. Exp. Biol. Med. 1993, im Druck) zu einem Hyperpolymeren mit sehr breiter Molekulargewichtsverteilung polymerisiert.

Für die Fällung wurde eine 4-molare (gesättigte) Ammoniumsulfat-Lösung hergestellt, deren pH-Wert mit 25%iger Amonniaklösung auf pH=7.3 eingestellt wurde. 0,8 ml einer 3,5%igen Lösung des o.g. Hämoglobin-Hyperpolymeren und 0,5ml der 4-molaren Ammoniumsulfatlösung wurden gemischt und 4,5 Stunden bei Raumtemperatur stehengelassen. Dann erfolgte scharfes Zentrifugieren und Abpipettieren des Überstandes.

Eine vergleichende Gelchromatographie an Sephacryl S-400 HR (Deutsche Pharmacia, Freiburg/D) zeigte an der unfraktionierten Probe ein unteres Molekulargewicht von 65 000 und ein oberes von 15 x 10⁶ g/mol. An der Probe im Überstand finden sich dagegen die Werte 65 000 bzw. 940 000 g/mol. Mit diesem Fällungsprozeß ist es somit gelungen, aus einem Hämoglobin-Hyperpolymerisat mit breitem Molekulargewichtsbereich die hochmolekularen Anteile zu entfernen.

### Beispiel 3 (Chromatographie)

Ausgangsmaterial ist ein gemäß Beispiel 2 hergestelltes DIBS-Polymeres. Die präparative Fraktionierung erfolgt an Sephacryl S-400 HR, Fließmittel HeNa (144 mmol/l NaCl; 10 mmol/l HEPES-Puffer, 0,2 g/l NaN₃), Säule: 2,6 cm Durchmesser, Füllvolumen 510 ml, Fluß 27 ml/h. Es wurden 10 ml einer 2,5%igen hyperpolymeren Hämoglobinlösung aufgetragen und die Fraktionen gelchromatographisch, wie in Beispiel 2 beschrieben, analysiert.
Der Bereich des Molekulargewichts der Ausgangsprobe lag zwischen 65 000 und 15 x 10⁶ g/mol; derjenige gewonnener Fraktionen lag beispielsweise zwischen 215 000 und 1,05 x 10⁶ g/mol sowie zwischen 65 000 und 0,32 x 10⁶ g/mol. Dieses Beispiel zeigt, daß sich chromatographisch einheitliche hyperpolymere Hämoglobine verschiedenen Molekulargewichts gewinnen lassen.

### Beispiel 4 (partielles Lösen)

Human-Hämoglobin wurde mit Glutardialdehyd, wie in Beispiel 1 beschrieben, vernetzt. Nach Teilen der Probe erfolgte (a) Lösen des Präzipitats für 24 Stunden und (b) Lösen für nur 30 Minuten. In beiden Fällen wurde der Überstand gewonnen und gelchromatographisch, wie in Beispiel 1 beschrieben, analysiert.
Im Fall (a) erstreckten sich die Molekulargewichte von 65 000 bis etwa 15 x 10⁶ g/mol, im Fall (b) dagegen von 65 000 bis 3,6 x 10⁶ g/mol.
Dieses Beispiel zeigt die Möglichkeit auf, durch die Zeitdauer des Lösens vorzugsweise Hämoglobin-Hyperpolymere niederen Molekulargewichts zu erhalten.

## Patentansprüche

1. Verfahren zur Herstellung molekular-einheitlicher hyperpolymerer Hämoglobine, dadurch gekennzeichnet,
daß eine an sich bekannte Lösung hyperpolymerer Hämoglobine unterschiedlichen Molekulargewichts wenigstens einem Ultrafiltrationsprozeß
und/oder wenigstens einem fraktionierten Fällungsprozeß
und/oder wenigstens einem Chromatographieprozeß
und/oder-wenigstens einem partiellen Lösungsprozeß unterworfen wird.

2. Verfahren nach Anspruch 1, dadurch gekennzeichnet,
daß bei dem bzw. den Ultrafilrationsprozeß (en) verschiedene Filtertypen verwendet werden,
und/oder eine Mehrfachfiltration stattfindet,
und/oder der pH-Wert geändert wird,
und/oder in verschiedenen Konzentrationen ultrafiltriert wird,
und/oder die Zusammensetzung des Lösungsmittels variiert wird,
und/oder die Temperatur variiert wird,
und/oder der transmembranöse Druck und/oder der tangentiale (Über-)Fluß variiert wird.

3. Verfahren nach Anspruch 1 oder 2, dadurch gekennzeichnet,
daß bei dem bzw. den fraktionierten Fällungsprozeß(en) verschiedene Agentien verwendet werden,
und/oder die Fällung bei verschiedenen Temperaturen erfolgt,
und/oder der pH-Wert variiert wird,
und/oder unterschiedliche Lösungsmittel für die zu fällenden polymeren Hämoglobine eingesetzt werden,
und/oder die Reaktionszeiten variiert werden,
und/oder unterschiedlich gesättigte Fällungsmittel verwendet werden.

4. Verfahren nach einem der Ansprüche 1 bis 3, dadurch gekennzeichnet,
daß bei dem bzw den Chromatographieprozeß(en) verschiedene chromatographische Verfahren, insbesondere Ionenaustausch- und Gelfiltrationschromatographie, kombiniert oder sukzessiv angewendet werden,
und/oder der pH-Wert geändert wird,
und/oder die Zusammensetzung des Fließmittels variiert wird,
und/oder die Chromatographiebedingungen, insbesonder der Fluß und die Beladung, variiert werden,
und/oder verschiedene Chromatographiematerialien eingesetzt werden.

5. Verfahren nach einem der Ansprüche 1 bis 4, dadurch gekennzeichnet,
daß bei dem bzw den partiellen Lösungsprozeß(en) die Lösungszeit variiert wird,
und/oder wenigstens eine Waschung durchgeführt wird,
und/oder die sich lösenden Polymere mit stabilisierenden Agenzien, die einen Abbau der Polymere insbesondere durch Oxidation verhindern, behandelt werden,
und/oder die Lösungsbedingungen, insbesondere die Temperatur und/oder die Menge
des Lösungsmittels, geändert werden,
und/oder die Zusammensetzung des Lösungsmittels variiert wird.
